(19) Europäisches Patentamt
European Patent Office
Office européen des brevets

(11) Veröffentlichungsnummer: **0 003 099**
**B1**

# (12) EUROPÄISCHE PATENTSCHRIFT

(45) Veröffentlichungstag der Patentschrift: **16.09.81**

(51) Int. Cl.³: **C 07 C 127/22**, C 07 C 149/437, A 01 N 37/26

(21) Anmeldenummer: **79100005.2**

(22) Anmeldetag: **02.01.79**

(54) **Substituierte N-Phenyl-N'-(2-chlor-6-fluor-benzoyl)-harnstoffe, Verfahren zu ihrer Herstellung und ihre Verwendung als Insektizide.**

(30) Priorität: **13.01.78 DE 2801316**

(43) Veröffentlichungstag der Anmeldung: **25.07.79 Patentblatt 79/15**

(45) Bekanntmachung des Hinweises auf die Patenterteilung: **16.09.81 Patentblatt 81/37**

(84) Benannte Vertragsstaaten: **BE CH DE FR GB IT NL SE**

(56) Entgegenhaltungen:
**DE-A-2 601 780**
**DE-A-2 820 696**

(73) Patentinhaber: **BAYER AG, Zentralbereich Patente, Marken und Lizenzen, D-5090 Leverkusen 1, Bayerwerk (DE)**

(72) Erfinder: **Sirrenberg, Wilhelm, Dr., Wuppertaler Strasse 21, D-4322 Sprockhövel (DE)**
Erfinder: **Klauke, Erich, Dr., Eichendorffweg 8, D-5068 Odenthal (DE)**
Erfinder: **Marhold, Albrecht, Dr., Carl-Duisberg-Strasse 329, D-5090 Leverkusen 1 (DE)**
Erfinder: **Hammann, Ingeborg, Dr., Belfortstrasse 9, D-5000 Köln 1 (DE)**
Erfinder: **Stendel, Wilhelm, Dr., In den Birken 55, D-5600 Wuppertal 1 (DE)**
Erfinder: **Krehan, Ingomar, Dr., Ludwig-Jahn-Strasse 54, D-5000 Köln 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Substituierte N-Phenyl-N'-(2-chlor-6-fluor-benzoyl)-harnstoffe, Verfahren zu ihrer Herstellung und ihre Verwendung als Insektizide

Die vorliegende Erfindung betrifft neue N-Phenyl-N'-(2-chlor-6-fluorbenzoyl)-harnstoffe, mehrere Verfahren zu ihrer Herstellung und ihre Verwendung als Insektizide.

Es ist bereits bekannt, dass bestimmte Benzoylharnstoffe, wie N-(2,6-Difluorbenzoyl)-N'-(4-chlorphenyl)-harnstoff, insektizide Eigenschaften besitzen [vergleiche J.Agr. Food Chem., Vol. 21, No. 6 (1973), S. 993–8]. Ebenso sind aus der DE-A-2 601 780 bestimmte N-Pehnyl-N'-(2,6-dihalogen-benzoyl)-harnstoffe bekannt, welche in der N-Penylgruppe durch Halogenalkoxy oder Halogenalkylthio substituiert sind. In allen vorbekannten 2,6-Dihalogen-benzoylverbindungen sind die 2,6-Positionen jeweils durch gleiche Halogenatome substituiert.

Es wurden die neuen N-Pehnyl-N'-(2-chlor-6-fluor-benzoyl)-harnstoffe der Formel I

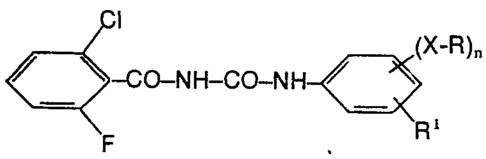

(I)

gefunden,
in welcher
R für Halogenalkyl mit 1 bis 4 Kohlenstoffatomen steht und
$R^1$ für Wasserstoff, Halogen oder Halogenalkyl steht und
X für Sauerstoff oder Schwefel steht und
n für die Zahl 1 oder 2 steht.
Diese neuen Verbindungen weisen starke insektizide Eigenschaften auf.

Weiterhin wurde gefunden, dass die neuen N-Phenyl-N'-(2-chlor-6-fluor-benzoyl)-harnstoffe der Formel I erhalten werden, wenn man
a) substituierte Aniline der Formel II

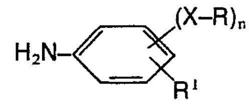

(II)

in welcher
R, $R^1$, X und n die oben angegebene Bedeutung haben, mit 2-Chlor-6-fluor-benzoylisocyanat, gegebenenfalls in Gegenwart eines Verdünnungsmittels, umsetzt oder
b) substituierte Phenylisocyanate der Formel III

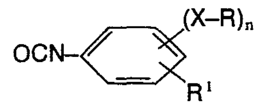

(III)

in welcher
R, $R^1$, X und n die oben angegebene Bedeutung haben,
mit 2-Chlor-6-fluor-benzamid, gegebenenfalls in Gegenwart eines Verdünnungsmittels, umsetzt.

Wie bereits oben erwähnt, sind die vorbekannten ähnlichen N-Phenyl-N'-(2,6-Dihalogen-benzoyl)-harnstoffe der DE-A-2 601 780 im Benzoylteil symmetrisch durch Halogen substituiert. Es war überraschend, dass ein Vergleich von symmetrisch substituierten Verbindungen des Standes der Technik gegenüber unsymmetrisch substituierten 2-Chlor-6-fluor-benzoyl-derivaten gemäss der vorliegenden Erfindung, eine bessere Wirksamkeit der neuen Verbindung ergab. Die Produkte gemäss vorliegender Erfindung stellen somit eine echte Bereicherung der Technik dar.

Verwendet man nach Verfahren (a) 3-Chlor-4-trifluormethoxyanilin und 2-Chlor-6-fluor-benzoylisocyanat und nach Verfahren (b) 3-Chlor-4-trifluormethoxy-phenylisocyanat und 2-Chlor-6-fluor-benzamid als Ausgangsstoffe, so kann der Reaktionsverlauf durch die folgenden Formelschemata wiedergegeben werden:

a)

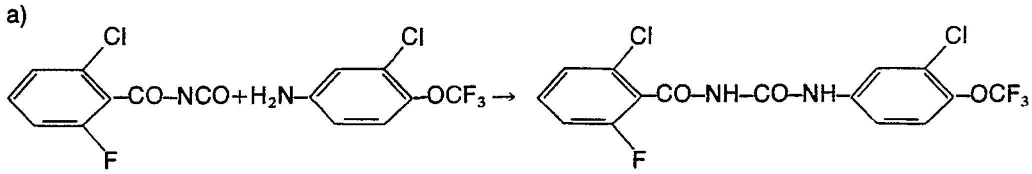

b)

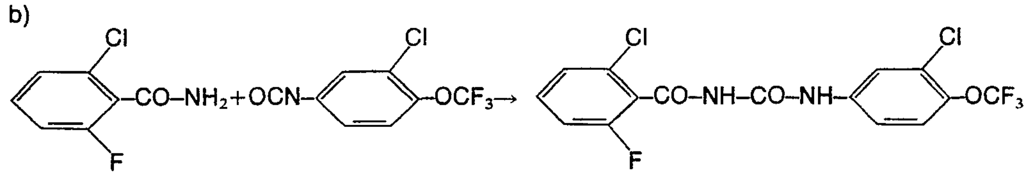

Die neuen Verbindungen sind durch die Formel (I) allgemein definiert.
Vorzugsweise stehen darin jedoch
R für geradkettiges oder verzweigtes Halogenalkyl mit 1 bis 4, insbesondere 1 oder 2 Kohlenstoffatomen und 1–4 Halogenatomen und
$R^1$ für Wasserstoff, Chlor oder Trifluormethyl.

Besonders erwähnt seien folgende Verbindungen der Formel I:
N-[2-Chlor-5-(2-chlor-1,1,2-trifluoräthoxy)]- phenyl-

N-[4-Chlor-3-(2-chlor-1,1,2-trifluoräthoxy)]-phenyl-
N-[3-(2-Chlor-1,1,2-trifluoräthoxy)]-phenyl-
N-[3-Chlor-4-(chlordifluormethoxy)]-phenyl-
N-[3-Chlor-4-(chlordifluormethylthio)]-phenyl-
N-[2-Chlor-4-(chlordifluormethoxy)]-phenyl-
N-[2-Chlor-4-(chlordifluormethylthio)]-phenyl-
N-[2-Chlor-5-(chlordifluormethoxy)]-phenyl-
N-[2-Chlor-5-(trifluormethylthio)]-phenyl-
N'-(2-Chlor-6-fluorbenzoyl)-harnstoff

Die als Ausgangsstoffe zu verwendenden substituierten Aniline der Formel (II) sind bekannt oder nach literaturbekannten Verfahren herstellbar [vergleiche z. B. J. Org. Chem. 25 (1960), 965 und 29 (1964), 1; J. Am. Chem. Soc. 73 (1951) 5831; Bull. Soc. Chim. France 4 (1957), 531; Z. obsc. Chim. 35 (1965), 1377 engl. Transl.; J. Am. Chem. Soc. 83 (1961), 4360 und US-Patentschrift 3 387 037]; die Aminogruppe kann nach üblichen Verfahren in die Isocyanatgruppe umgewandelt werden, z.B. durch Umsetzung mit Phosgen, wodurch die entsprechenden Isocyanate der Formel (III) erhalten werden.

Diese Ausgangsstoffe sind durch die Formeln II und III allgemein definiert. R und $R^1$ in diesen Formeln besitzen darin vorzugsweise die bei den Endprodukten der Formel I angegebene Definition.

Als Beispiele seien im einzelnen genannt:
4-Difluormethoxy-, 4-Difluormethylthio-, 3-Difluormethoxy-, 3-Difluormethylthio-, 2-Difluormethoxy-, 2-Difluormethylthio-, 4-Trifluormethoxy-, 4-Trifluormethylthio-, 3-Trifluormethoxy-, 3-Trifluormethylthio-, 2-Trifluormethoxy-, 2-Trifluormethylthio-, 4-Chlordifluormethoxy-, 4-Chlordifluormethylthio-, 3-Chlordifluormethoxy-, 3-Chlordifluormethylthio-, 2-Chlordifluormethoxy-, 2-Chlordifluormethylthio-, 3-Trifluormethyl-4-trifluormethoxy-, 3-Trifluormethyl-4-trifluormethylthio-, 3-Trifluormethyl-4-difluormethoxy-, 3-Trifluormethyl-4-trifluormethylthio-, 3,4-Bis-(difluormethoxy)-, 3,4-Bis-(difluormethylthio)-, 3,4-Bis-(trifluormethoxy)-, 3,4-Bis-(trifluormethylthio-, 2-Chlor-4-trifluormethoxy-, 2-Chlor-4-trifluormethylthio-, 3-Chlor-4-trifluormethoxy-, 3-Chlor-4-trifluormethylthio-, 2-Chlor-4-chlordifluormethoxy-, 2-Chlor-4-chlordifluormethylthio-, 3-Chlor-4-chlordifluormethoxy-, 3-Chlor-4-chlordifluormethylthio-, 2-(1,1,2,2-tetrafluoräthoxy), 3-(1,1,2,2-tetrafluoräthoxy)-, 4-(1,1,2,2-tetrafluoräthoxy)-, 2-Chlor-4-(1,1,2,2-tetrafluoräthoxy)-, 3-Chlor-4-(1,1,2,2-tetrafluoräthoxy)-, 4-(2-chlor-1,1,2-trifluoräthoxy)-, 2-Chlor-4-(2-chlor-1,1,2-trifluoräthoxy)-, 3-Chlor-4-(2-chlor-1,1,2-trifluoräthoxy)- und 4-(2-Chlor-1,1,2-trifluoräthylthio)-anilin bzw. -phenylisocyanat.

2-Chlor-6-fluor-benzoylisocyanat bzw. 2-Chlor-6-fluorbenzamid können als Ausgangsstoffe des erfindungsgemässen Syntheseverfahrens ebenfalls nach literaturbekannten Methoden hergestellt werden [vergleiche J. Org. Chem. 30 (1965), 4306 bzw. Beilstein «Handbuch der organischen Chemie» Band 9, S. 336].

Die Verfahren zur Herstellung der erfindungsgemässen N-Phenyl-N'-benzoyl-harnstoffe werden bevorzugt unter Verwendung geeigneter Verdünnungsmittel durchgeführt. Als solche kommen praktisch alle inerten organischen Solventien in Frage. Hierzu gehören insbesondere aliphatische und aromatische, gegebenenfalls chlorierte, Kohlenwasserstoffe wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Die Reaktionstemperatur kann innerhalb eines grösseren Bereichs variiert werden. Im allgemeinen arbeitet man zwischen 0 und 120 °C, vorzugsweise bei 70 bis 85 °C.

Die Umsetzung lässt man im allgemeinen bei Normaldruck ablaufen.

Zur Durchführung des Verfahrens setzt man die Ausgangskomponenten vorzugsweise in äquimolaren Verhältnissen ein. Ein Überschuss der einen oder anderen Reaktionskomponente bringt keine wesentlichen Vorteile.

Im allgemeinen gibt man die Reaktionspartner in einem der angegebenen Lösungsmittel zusammen. Die bei der Reaktionsvariante (b) einzusetzenden Phenylisocyanate (III) können in reiner Form oder in Form ihrer Reaktionsmischung, die nach der Umsetzung des entsprechenden Anilins mit Phosgen erhalten wird, ohne Isolierung eingesetzt werden. Diese Reaktionsmischung wird vorzugsweise in einem der oben angegebenen Lösungsmittel mit 2-Chlor-6-fluor-benzamid versetzt.

Die Reaktionen werden unter den oben angegebenen Bedingungen durchgeführt und die Produkte durch Filtration isoliert. Die neuen Verbindungen fallen in kristalliner Form mit scharfem Schmelzpunkt an.

Wie bereits mehrfach erwähnt, zeichnen sich die erfindungsgemässen N-Phenyl-N'-(2-Chlor-6-fluor-benzoyl)-harnstoffe durch eine hervorragende insektizide Wirksamkeit aus. Sie wirken auch auf dem veterinär-medizinischen Sektor gegen tierische Parasiten (Ektoparasiten), wie parasitierende Fliegenlarven.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B.. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aëdes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnussschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z. B. Alkylaryl-polyglykoläther, Alkylsulfonate, Alkylsulfate, Arylsulfonate sowie Eiweisshydrolysate; als Dispergiermittel: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabikum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel A

Plutella-Test

Lösungsmittel: 3 Gewichtsteile Dimethylformamid
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z. B. die Verbindungen der Herstellungsbeispiele 1, 2, 5, 11, 12, 13, 17 und 18 eine besonders günstige Wirkung.

Beispiel B

Mückenlarven-Test

Testtiere: Aedes aegypti
Lösungsmittel: Aceton 99 Gewichtsteile
Emulgator: 1 Gewichtsteil

Zur Herstellung einer Alkylarylpolyglykoläther zweckmässigen Wirkstoffzubereitung löst man 2 Gewichtsteile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wässrigen Wirkstoffzubereitungen der gewünschten Konzentration in Gläser und setzt anschliessend etwa 25 Mückenlarven in jedes Glas ein.

Nach 24 Stunden wird der Abtötungsgrad in % bestimmt. Dabei bedeutet 100%, dass alle Larven abgetötet worden sind. 0% bedeutet, dass überhaupt keine Larven abgetötet worden sind.

Bei diesem Test zeigen z. B. die Verbindungen der Herstellungsbeispiele 1, 12 und 17 eine besonders günstige Wirkung.

Beispiel C

Test mit parasitierenden Fliegenlarven

Lösungsmittel: 80 Gewichtsteile Alkylaryl

Zur Herstellung einer zweckmässigen Wirkstoffzubereitung vermischt man 20 Gewichtsteile der betreffenden aktiven Substanz mit der angegebenen Menge Lösungsmittel und verdünnt das so erhaltene Konzentrat mit Wasser auf die gewünschte Konzentration.

Etwa 20 Fliegenlarven (Lucilia cuprina) werden in ein Teströhrchen gebracht, welches ca. 2 $cm^3$ Pferdemuskulatur enthält. Auf dieses Pferdefleisch werden 0,5 ml der Wirkstoffzubereitung gebracht. Nach 24 Stunden wird der Abtötungsgrad in % bestimmt. Dabei bedeuten 100%, dass alle, und 0% dass keine Larven abgetötet worden sind.

Bei diesem Test zeigen z. B. die folgenden Verbindungen der Herstellungsbeispiele eine besonders günstige Wirkung: 1, 12, 17.

Herstellungsbeispiele

Beispiel 1

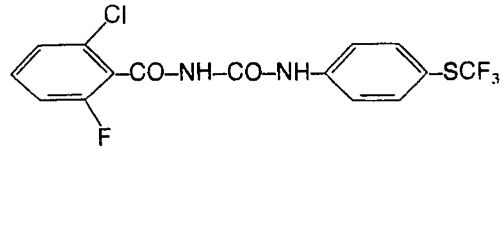

3,87 g (0,02 Mol) 4-Trifluormethylmercaptoanilin werden in 50 ccm trockenem Toluol gelöst. Dazu fügt man bei 60 °C eine Lösung von 3,99 g (0,02 Mol) 2-Chlor-6-fluor-benzoylisocyanat in 15 ccm Toluol. Der Ansatz wird 1 Stunde bei 80 °C gerührt und dann auf Raumtemperatur abgekühlt. Nach dem Trocknen erhält man 5,4 g (69% der Theorie) an N-(4-Trifluormethylmercaptophenyl)-N'-(2-chlor-6-fluor-benzoyl)-harnstoff. Das Produkt hat einen Schmelzpunkt von 202 °C.

In analoger Arbeitsweise wurden auch die anderen Beispiele hergestellt, wobei eine Optimierung der Ausbeuten nicht erfolgte, aber möglich ist. Die Reinheit sowie Identität der Produkte wurde durch Elementaranalyse und NMR-Spektren sichergestellt.

Allgemeine Formel

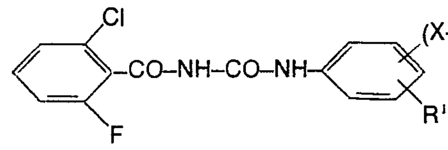

(I)

| Beispiel Nr. | R | R' | n | X | Schmelzpunkt (°C) | Ausbeute (% der Theorie) |
|---|---|---|---|---|---|---|
| 2 | 4-$CF_3$ | H | 1 | 0 | 180 | 63,5 |
| 3 | 3-$CF_3$ | H | 1 | 0 | 162 | 67,5 |
| 4 | 2-$CF_3$ | H | 1 | 0 | 154 | 67,5 |
| 5 | 4-$CF_3$ | 3-Cl | 1 | 0 | 189 | 74 |
| 6 | 4-$CF_3$ | 2-Cl | 1 | 0 | 221 | 95,5 |
| 7 | 4-$CHF_2$ | H | 1 | 0 | 173–4 | 83,5 |
| 8 | 3-$CHF_2$ | H | 1 | 0 | 132 | 72,5 |
| 9 | 3,4-$CHF_2$ | H | 2 | 0 | 195 | 89 |
| 10 | 4-$CHF_2$ | 3-$CF_3$ | 1 | 0 | 221 | 84,5 |
| 11 | 4-$CF_2Cl$ | H | 1 | 0 | 203 | 82,5 |
| 12 | 4-$CF_2$–CHFCl | H | 1 | 0 | 199 | 83,5 |
| 13 | 4-$CF_2$–CHFCl | 3-Cl | 1 | 0 | 178 | 70,5 |
| 14 | 4-$CF_2$–$CHF_2$ | 3-Cl | 1 | 0 | 176 | 90,0 |
| 15 | 3-$CF_2$–$CHF_2$ | H | 1 | 0 | 185 | 69,5 |
| 16 | 3-$CF_3$ | H | 1 | S | 172 | 43 |
| 17 | 4-$CF_3$ | 3-Cl | 1 | S | 187 | 54 |
| 18 | 4-$CF_2Cl$ | 3-Cl | 1 | S | 176 | 60,5 |
| 19 | 4-$CF_2$CHFCl | H | 1 | S | 189 | 91,5 |

Die erfindungsgemässen Verbindungen lassen sich auch durch Umsetzung von 2-Chlor-6-fluorbenzamid mit den entsprechenden Isocyanaten herstellen.

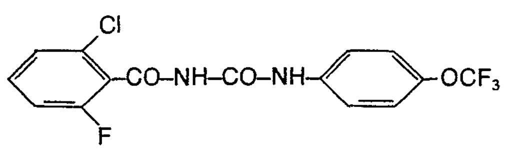

5,02 g (0,03 Mol) 2-Chlor-6-fluor-benzamid werden in 50 ccm trockenem Toluol suspendiert. Dazu fügt man eine Lösung von 6,1 g (0,03 Mol) 4-Trifluormethoxy-phenylisocyanat in 10 ccm Toluol und kocht den Ansatz 5 Stunden unter Rückfluss. Danach kühlt man den Ansatz auf Raumtemperatur ab und saugt das ausgefallene Produkt ab. Es wird aus Toluol umkristallisiert und zeigt einen Schmelzpunkt von 178 °C. Reinheit und Identität wurden durch Elementaranalyse und NMR-Spektrum sichergestellt. Das NMR-Spektrum ist mit dem Spektrum der auf anderem Wege hergestellten Substanz identisch.

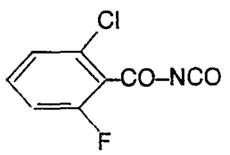

2-Chlor-6-fluor-benzoylisocyanat wurde nach literaturbekanntem Verfahren hergestellt [vergleiche Speziale et al. J. Org. Chem. 30 (12) S. 4306–7 (1965)]. Es besitzt einen Siedepunkt von 75 °C/1 Torr.

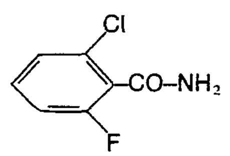

Das 2-Chlor-6-fluor-benzamid wurde nach literaturbekannten Verfahren aus dem Säurechlorid mit Ammoniak hergestellt. Es hat einen Schmelzpunkt von 138 °C.

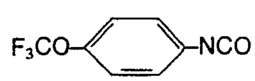

4-Trifluormethoxy-phenylisocyanat wurde aus dem entsprechenden Anilin mit Phosgen nach literaturbekanntem Verfahren hergestellt mit einem Siedepunkt von 37 °C/1 Torr und einem Brechungsindex von $n_D^{20}$: 1,4600.

**Patentansprüche**

1. N-Phenyl-N'-(2-chlor-6-fluorbenzoyl)-harnstoffe der allgemeinen Formel I

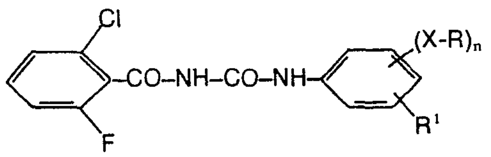

(I)

in welcher

R für Halogenalkyl mit 1–4 C-Atomen steht und

R[1] für Wasserstoff, Halogen oder Halogenalkyl mit 1–4 C-Atomen steht und

X für Sauerstoff oder Schwefel steht und

n für 1 oder 2 steht.

2. Verfahren zur Herstellung der N-Phenyl-N'-(2-chlor-6-fluorbenzoyl)-harnstoffe gemäss Anspruch 1, dadurch gekennzeichnet, dass man

a) substituierte Aniline der Formel II

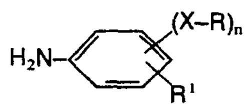

(II)

in welcher

R, R[1], X und n die oben angegebene Bedeutung haben,

mit 2-Chlor-6-fluor-benzoylisocyanat, gegebenenfalls in Gegenwart eines Verdünnungsmittels umsetzt oder

b) substituierte Phenylisocyanate der Formel III

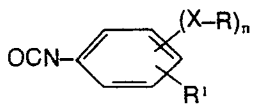

(III)

in welcher

R, R[1], X und n die oben angegebene Bedeutung haben,

mit 2-Chlor-6-fluor-benzamid, gegebenenfalls in Gegenwart eines Verdünnungsmittels, umsetzt.

3. Insektizide Mittel, gekennzeichnet durch einen Gehalt an mindestens einem N-Phenyl-N'-(2-chlor-6-fluorbenzoyl)-harnstoff gemäss Anspruch 1.

4. Verwendung von N-Phenyl-N'-(2-chlor-6-fluorbenzoyl)-harnstoffen gemäss Anspruch 1 zur Bekämpfung von Insekten.

5. Verfahren zur Herstellung insektizider Mittel, dadurch gekennzeichnet, dass man N-Phenyl-N'-(2-chlor-6-fluorbenzoyl)-harnstoffe gemäss Anspruch 1 mit Streckmitteln und/oder oberflächenaktiven Mitteln vermischt.

**Claims**

1. N-Phenyl-N'-(2-chloro-6-fluorobenzoyl)-ureas of the general formula I

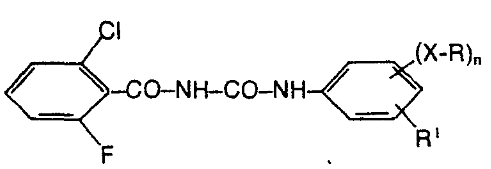

(I)

in which

R represents halogenoalkyl with 1–4 C atoms,

R[1] represents hydrogen, halogen or halogenalkyl with 1–4 C atoms,

X represents oxygen or sulphur and

n represents the number 1 or 2.

2. Process for the preparation of the N-phenyl-N'-(2-chloro-6-fluoro-benzoyl)-ureas according to Claim 1, characterised in that

a) substituted anilines of the formula II

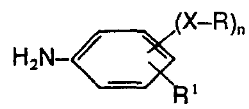

(II)

in which

R, R[1], X and n have the meaning indicated above, are reacted with 2-chloro-6-fluoro-benzoyl isocyanate, optionally in the presence of a diluent, or

b) substituted phenyl isocyanates of the formula III

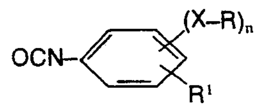

(III)

in which

R, R[1], X and n have the meaning indicated above, are reacted with 2-chloro-6-fluoro-benzamide, optionally in the presence of a diluent.

3. Insecticidal agents, characterised in that they contain at least one N-phenyl-N'-(2-chloro-6-fluorobenzoyl)-urea according to Claim 1.

4. Use of N-phenyl-N'-(2-chloro-6-fluorobenzoyl)-ureas according to Claim 1 for combating insects.

5. Process for the preparation of insecticidal agents, characterised in that N-phenyl-N'-(2-chloro-6-fluorobenzoyl)-ureas according to Claim 1 are mixed with extenders and/or surface-active agents.

**Revendications**

1. N-Phényl-N'-(2-chloro-6-fluorobenzoyl)-urées de formule générale (I):

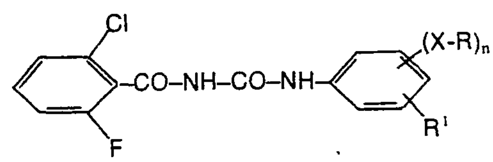

(I)

dans laquelle:

R représente un groupe halogénoalkyle en $C_1$–$C_4$ et

R[1] représente l'hydrogène, un halogène ou un groupe halogénoalkyle en $C_1$–$C_4$ et

X représente l'oxygène ou le soufre et

n est égal à 1 ou 2.

2. Procédé de préparation des N-Phényl-N'-(2-chloro-6-fluorobenzoyl)-urées selon la revendication 1, caractérisé en ce que:

a) on fait réagir des anilines substituées de formule (II):

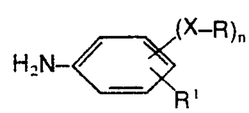

(II)

dans laquelle R, R', X et n ont les significations indiquées ci-dessus, avec le 2-chloro-6-fluorobenzoylisocyanate, éventuellement en présence d'un diluant, ou bien

b) on fait réagir des phénylisocyanates substitués de formule (III):

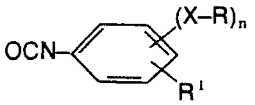

(III)

dans laquelle R, R', X et n ont les significations indiquées ci-dessus, avec le 2-chloro-6-fluorobenzamide, éventuellement en présence d'un diluant.

3. Produit insecticide, caractérisé en ce qu'il contient au moins une N-phényl-N'-(2-chloro-6-fluorobenzoyl)-urée selon la revendication 1.

4. Utilisation des N-phényl-N'-(2-chloro-6-fluorobenzoyl)-urées selon la revendication 1 dans la lutte contre les insectes.

5. Procédé de préparation de produits insecticides, caractérisé en ce que l'on mélange des N-phényl-N'-(2-chloro-6-fluorobenzoyl)-urées selon la revendication 1 avec des diluants et/ou agents tensio-actifs.